# EUROPÄISCHE PATENTSCHRIFT

(11) **EP 1 041 896 B1**
(45) Veröffentlichungstag und Bekanntmachung des Hinweises auf die Patenterteilung: **20.02.2002**
(21) Anmeldenummer: 98966662.3
(22) Anmeldetag: 22.12.1998
(51) Int. Cl.: A23L 1/30, A23D 9/00, A61K 31/20

(54) **FETTMISCHUNG UND DIESELBEN ENTHALTENDES DIÄTEISCHES ODER PHARMAZEUTISCHES MITTEL**
FAT BLEND
MELANGE DE CORPS GRAS

(30) Priorität: 23.12.1997 DE 19757414
(43) Veröffentlichungstag der Anmeldung: 11.10.2000
(73) Patentinhaber: N.V. Nutricia, 2712 HM Zoetermeer (NL)
(72) Erfinder: SAWATZKI, Günther, D-35516 Münzenberg (DE); BOEHM, Günther, D-61209 Echzell (DE); KOHN, Gerhard, D-55268 Nieder-Olm (DE); FARWER, Sandra, D-91056 Erlangen (DE); KLIEM, Michael, D-91086 Aurachtal (DE)
(74) Vertreter: Köster, Hajo, Dr.
(86) Internationale Anmeldenummer: EP9808409
(87) Internationale Veröffentlichungsnummer: WO9933355

(56) Entgegenhaltungen:
- EP-A- 0 615 753
- EP-A- 0 682 878
- EP-A- 0 756 827
- EP-A- 0 764 405
- DE-A- 3 924 607
- US-A- 5 223 285

## Beschreibung

Die Erfindung betrifft eine Fettmischung auf Basis von Ölen, Fetten und/oder Lezithinen mit mehrfach ungesättigten Fettsäuren, ein diese Fettmischung enthaltendes diätetisches oder pharmazeutisches Mittel und die Verwendung dieser Fettmischung bzw. dieses diätetischen oder pharmazeutischen Mittels.

Der Körper ist bekanntlich in der Lage, bestimmte gesättigte und einfach ungesättigte Fettsäuren einschließlich der Stearinsäure (C18-0) und der Ölsäure (C18-1w9) endogen zu synthetisieren. Der Körper ist jedoch nicht in der Lage, die für ihn erforderlichen polyungesättigten Fettsäuren Linolsäure (18-2w6) und alpha-Linolensäure (C18-3w3) endogen zu synthetisieren, so daß diese Fettsäure exogen mit der Nahrung zugeführt werden müssen und daher auch als essentielle Fettsäuren bezeichnet werden.

Aus diesen essentiellen Fettsäuren werden dann im menschlichen Fettsäurestoffwechsel durch Kettenverlängerung (Elongation) und Desaturierung eine Vielzahl von längerkettigen (C20 und C22) und höher desaturierten Fettsäuren synthetisiert. Bei den Fettsäuren, die sich von der Linolsäure (C18-2w6) ableiten, spricht man von der w6-Familie, während sich von der alpha-Linolensäure die w3-Familie ableitet. Diese mehrfach ungesättigten Fettsäuren werden im englischen auch als polyunsaturated fatty acids oder PUFA bezeichnet. Bezüglich der im Rahmen der vorliegenden Unterlagen verwendeten Kurzbezeichnung bzw. Nomenklatur wird im übrigen ergänzend verwiesen auf "lipid analysis" von William W. Christie, Pergamon Press 1973.

Die genannten mehrfach ungesättigten Fettsäuren sind strukturelle Bestandteile jeglicher Zellmembranen des Körpers. Einigen speziellen Fettsäuren aus der w3- und der w6-Familie kommt besondere Bedeutung zu, da aus ihnen spezielle Moleküle synthetisiert werden, die in ihrer Gesamtheit als Eicosanoide bezeichnet werden.

Unter Eicosanoiden als Sammelbegriff versteht man heute eine außerordentlich vielfältige und komplexe Mischung von physiologisch hochwirksamen, hormonähnlichen Verbindungen, die an vielfältigen Regulationsvorgängen im Körper teilnehmen. Die Eicosanoide leiten sich vor allem aus den w6- und w3-desaturierten C20-Präkursorfettsäuren dihomo-gamma-Linolensäure (DGLA; 20-3w6), Arachidonsäure (AA; 20-4w6), Eicosatetraensäure (20-4w3) und Eicosapentaensäure (EPA; 20-5w3) ab.

Die biologischen Effekte der aus den mehrfach ungesättigten Fettsäuren gebildeten Eicosanoide unterscheiden sich außerordentlich und je nach dem, ob sich die Eicosanoide von den Fettsäuren der w6- oder w3-Familie ableiten. Im Allgemeinen werden den Eicosanoiden der w3-Reihe anti-inflammatorische Effekte zugeschrieben, während die Eicosanoide der Arachidonsäure aus der w6-Familie pro-inflammatorischen Charakter besitzen.

Aufgrund der Ernährungspraktiken und Ernährungsweise, insbesondere in den westlichen Ländern, kommt es nun zu einer Erhöhung der Arachidonsäuregehalte in den Membranlipiden der Körperzellen und damit zu einer erhöhten Synthese der von der Arachidonsäure abzuleitenden, pro-inflammatorischen Eicosanoide.

In der letzten Zeit wurde nun versucht, durch die gezielte diätetische Aufnahme von speziellen mehrfach ungesättigten Fettsäuren die Krankheitsbilder verschiedener chronisch-entzündlicher Erkrankungen sowie von Lipidstoffwechselstörungen positiv zu beeinflussen. So beschreiben beispielsweise die EP-A 0 756 827 und die EP-A 0 764 405 die Verabreichung von Fettblends bzw. von Fettmischungen auf Basis von Nachtkerzenöl und/oder Fischöl zur Modulation des Immunsystems. Die DE-A 39 24 607 empfiehlt die Verwendung diätetischer Produkte auf Basis von Fischöl zur Blutdrucksenkung bei Hyperlipidämien. In der EP-A 0 457 950 wird darüber hinaus die Verwendung von Stearidonsäure in pharmazeutischen Zusammensetzungen zur Behandlung von Krankheiten mit entzündlichem Ursprung beschrieben.

Auf dem Markt werden auch bereits Fettemulsionen zur enteralen Ernährung angeboten, die als wesentliche Fettsäuren gamma-Linolensäure (GLA), Eicosapentaensäure (EPA) und zum Teil auch Stearidonsäure (SA) enthalten, welche zu einer Immunmodulation dienen sollen.

Bei allen den in den aufgeführten Druckschriften beschriebenen Produkten und auch bei den auf dem Markt erhältlichen Produkten stehen die zur Anwendung gebrachten mehrfach ungesättigten Fettsäuren jedoch in einem unausgewogenen Verhältnis zueinander.

Aufgabe der vorliegenden Erfindung ist es, eine verbesserte Fettmischung und ein diese enthaltende diätetisches oder pharmazeutisches Mittel bereitzustellen, mit der der Fettsäurestoffwechsel und insbesondere der Eicosanoidstoffwechsel in optimaler Weise beeinflußt werden kann, so daß durch die Verabreichung dieser Fettmischung bzw. Nahrung die Symptomatik und die klinischen Probleme von Patienten mit verschiedenen Erkrankungen signifikant verbessert werden.

Gelöst wird diese Aufgabe durch eine Fettmischung und eine diese Fettmischung enthaltendes diätetisches Nahrungsmittel gemäß der Lehre der Ansprüche.

Es wurde nämlich überraschend festgestellt, daß der Eicosanoidstoffwechsel der Arachidonsäure effektiv und optimiert dadurch beeinflußt werden kann, daß die mehrfach ungesättigten Fettsäuren gamma-Linolensäure (GLA), Eicosapentaensäure (EPA) und Stearidonsäure (SA)in einem spezifischen, balancierten Verhältnis zueinander verabreicht werden. Beansprucht wird daher, daß in der Fettmischung die GLA und die EPA jeweils 20 bis 50 Gew.-% und die SA 15 bis 50 Gew-% der aus diesen drei Fettsäuren gebildeten Summe ausmachen. Zudem macht die Summe dieser Fettsäuren zusammen 10 bis 500 mg pro g der Gesamtfettsäure (Summe der insgesamt vorhandenen Fettsäuren) aus.

Werden die drei genannten Fettsäuren in der beanspruchten Menge und in den beanspruchten Verhältnissen verabreicht, dann wird die Entstehung von pro-inflammatorischen Eicosanoiden der Arachidonsäure negativ beeinflußt. Zudem wird das physiologische Gleichgewicht der Eicosanoide im Hinblick auf eine anti-inflammatorische und lipidsenkende Wirkung verschoben. Auch kann durch die erfindungsgemäße Fettmischung eine entwicklungs- oder krankheitsbedingte Verminderung der lipolytischen Kapazität des Gastrointestinaltraktes stimmuliert und verbessert werden.

Erfindungsgemäß wird somit eine Fettmischung bereitgestellt, die sich durch hohe Gehalte und ein spezifisches Verhältnis an bestimmten mehrfach ungesättigten Fettsäuren zueinander auszeichnet. Diese Fettmischung oder ein dieses enthaltendes Nahrungsmittel kann an Patienten mit akut- und chronisch-inflammatorischen Erkrankungen, an Patienten mit Autoimmunerkrankungen, an Patienten mit Fettstoffwechselstörungen (Hyperlipidämien), an Patienten mit geschwächter Immunfunktion und an Patienten mit eingeschränkter lipolytischer Kapazität des Gastrointestinaltraktes verabreicht werden. Weitere Anwendungsgebiete der erfindungsgemäß beanspruchten Gegenstände sind weiter unten näher erläutert.

Bei der erfindungsgemäßen Fettmischung liegen die Fettsäuren vorzugsweise in derjenigen Form vor, in der sie im zur Anwendung gebrachten Rohstoff Öl, Fett und Lezithin gebunden sind, d.h. insbesondere als Triglyceride und Phospholipide. Diese Fettsäuren können jedoch insgesamt oder teilweise auch als freie Fettsäuren, als Ester, beispielsweise einfache Alkylester wie Ethylester, oder als Salz vorliegen. Auch ist es möglich, umgeesterte Fettsäuren zum Einsatz zu bringen. So kann die erfindungsgemäße Mischung beispielsweise mit derartigen freien Fettsäuren, einfachen Fettsäureestern und Fettsäuresalzen supplementiert sein. Es ist sogar erfindungsgemäß umfaßt, daß die erfindungsgemäße Mischung ausschließlich aus diesen freien Fettsäuren, einfachen Fettsäureestern und/oder Fettsäuresalzen besteht und daher terminologisch an sich auch als Fettsäuremischung zu bezeichnen wäre.

Die erfindungsgemäße Fettmischung enthält zweckmäßigerweise verschiedene Öle, Fette und/oder Lezithine. So kann die Fettmischung beispielsweise verschiedene derartige Öle, Fette und Lezithine enthalten, die keine oder nur geringe Gehalte an mehrfach ungesättigten Fettsäuren aufweisen. Um letztere Fettsäuren dann in die Fettmischung zu inkorporieren, werden diese Öle, Fette und/oder Lezithine mit solchen gemischt, welche eben die mehrfach ungesättigten Fettsäuren besitzen.

Bei den Ölen, Fetten und/oder Lezithinen kann es sich um übliche handeln, beispielsweise tierische und pflanzliche. Allerdings können auch Öle, Fette und Lezithine mikrobiellen und/oder synthetischen Ursprungs und somit auch neu entwickelte Ausgangsstoffe eingesetzt werden. Auch erst noch in der Zukunft zu entwickelnde Rohstoffe können eingesetzt werden, denn bezüglich der zur Anwendung gebrachten Ausgangsstoffe kommt es lediglich darauf an, daß sie die spezifizierten Fettsäuren in den aufgeführten Mengen und Verhältnissen enthalten.

Nach einer bevorzugten Ausführungsform machen die Fettsäuren GLA, SA und EPA zusammen 10 bis 100 mg pro g der ingesamt vorhandenen Fettsäuren aus; zudem machen die GLA und die EPA jeweils 35 bis 45 (z.B. 40) Gew.-% und die SA 15 bis 25 (z.B. 20) Gew.-% der Summe dieser drei Fettsäuren aus. Wenn im Rahmen der vorliegenden Unterlagen von einem Bereich die Rede ist, dann sind alle die in diesem Bereich fallenden Zwischenwerte offenbart. Somit stellt die Schreibweise 10 bis 100 mg bzw. 10 bis 500 mg nur eine verkürzte Schreibweise für alle dazwischenliegenden Werte, insbesondere alle ganzzahligen Werte dar, beispielsweise 10, 11, 12, 13, 15,...30, 31, 32, 33...65, 66, 67, 68,...85,...104, 105, 106,...150, 151, 152,...187, 188, 189, 190,...215, 216, 217,...241, 242, 243,...268, 269, 270,...280,...290,...300, 301, 302, 303, 304..., 310..., 320..., 330..., 340..., 350..., 360, 361..., 370..., 380..., 390..., 400..., 410..., 415, 416, 417..., 420..., 430..., 440..., 450..., 460..., 470..., 480..., 490, 491..., 495, 496.... Entsprechendes gilt für die Gewichtsprozentbereiche von 15 bis 50 Gew.-%, 35 bis 45 Gew.-% und 15 bis 25 Gew.-%. Dadurch sind zumindest alle dazwischenliegenden ganzzahligen Werte offenbart, beispielsweise 15, 18, 21, 24, 27, 28, 31, 33, 37, 39, 40, 42, 44, 47 und 49. Zudem sind auch alle von den größeren Bereichen umfaßten kleineren Bereiche mit umfaßt.

Das oben Gesagte gilt auch bezüglich der in den vorliegenden Unterlagen beanspruchten Fettgehalte in Form von Energie-% und für die beanspruchten Gewichtsprozentangaben für die Lezithine. Auch hier sind insbesondere alle ganzzahligen Werte zwischen den Grenzwerten dieser Bereiche offenbart.

Nach einer bevorzugten Ausführungsform enthält die Fettmischung auch Arachidonsäure (AA). Der Quotient aus der Summe aus GLA +SA+EPA zur AA beträgt dabei mindestens 10:1.

Nach einer weiterhin bevorzugten Ausführungsform beträgt der Lezithingehalt bis zu 40 Gew.-% der Gesamtlipide (= Summe der Öle, Fette und Lezithine), vorzugsweise 1 bis10 Gew.-%.

Nach einer weiterhin bevorzugten Ausführungsform macht die Summe der in der Fettmischung in Form von Phospholipiden vorhandenen Fettsäuren GLA, SA und EPA bis zu 120 mg / g der Gesamtfettsäuren, vorzugsweise 0,05 bis 50 mg pro g der Gesamtfettsäuren aus. Diese in Form von Phospholipiden vorliegenden Fettsäuren können somit beispielsweise 0,05, 0,1, 0,5, 1, 2, 3, 4, 5, 6, 7, 8, 9 und 10 mg pro g Gesamtfettsäuren ausmachen. Auch in diesem Fall sind wieder alle zwischen den Grenzwerten liegenden Bereiche offenbart.

Wie bereits dargelegt, kann man die erfindungsgemäße Fettmischung herstellen indem man tierische, pflanzliche, mikrobieile und/oder synthetische Öle, Fette und/oder Lezithine in bestimmten Mengenanteilen miteinander vermischt.

Als pflanzliche Öle können zum Beispiel "konventionelle" Öle aus mono- und dicotylen Pflanzen (wie z.B. Kokosöl, Palmkernöl, Palmöl, Soyaöl, Sonnenblumenöl, Rapsöl) verwendet werden. Zur gezielten Erhöhung des gamma-Linolen-(GLA)- und Stearidonsäure-(SA)-Gehaltes werden "spezielle" pflanzliche Öle wie Borretschöl, Nachtkerzenöl, Echiumöl, Trichodesmaöl, sowie die Samenöle weiterer Spezies z.B. aus den Familen der Boraginaceae, Scrophulariaceae, Onagraceae und Saxifragaceae eingesetzt. Außerdem können beispielsweise auf chemischem oder enzymatischem Wege hergestellte und auch durch chromatografische Auftrennung erhaltene GLA- und SA-reiche Konzentrate aus den genannten Quellen verwendet werden. Als tierische Fette und Öle können beispielsweise Eieröle, Fischöle und Öle von marinen Säugetieren, sowie beispielsweise auf chemischem bzw. enzymatischem Wege hergestellte oder auch durch chromatografische Trennung erhaltene eicosapentaensäurereiche und/oder stearidonsäurereiche Konzentrate aus diesen Rohstoffen eingesetzt werden. Weiterhin können gamma-Linolen-, Stearidon- sowie Eicosapentaensäure enthaltende Öle und Fette aus mikrobiellem Ursprung bzw. entsprechende Algen- und Pilzöle sowie daraus abzuleitende Konzentrate verwendet werden.

Weiterhin können in der erfindungsgemäßen Fettmischung spezifische GLA-, SA- und EPA-enthaltende Lezithine eingesetzt werden; hierunter sind zu nennen Lezithine aus Eigelb, vorzugsweise solche, die durch eine modifizierte Fütterung ein w3-PUFA betontes Fettsäurespektrum aufweisen, außerdem andere natürliche w3-PUFA-enthaltende Lezithine, beispielsweise aus Fischen, marinen Säugetieren oder aus Mikroorganismen sowie solche Lezithine, die auf chemischem oder enzymatischem Wege in ihrem Gehalt an GLA, SA und EPA, vorzugsweise in der sn-2-Stellung am Glyceringerüst, angereichert wurden. Weiterhin können in der beanspruchten Fettmischung mittelkettige Triglyceride (MCT) eingesetzt werden. Die hier verwendeten Ausdrücke "Fette, Öle und Lezithine" bezeichnen technologische Ausgangsprodukte. Bezeichnungen wie Phospholipide und Triglyceride beziehen sich hingegen auf die chemischen Spezies. So kann ein Öl durchaus auch Phospholipide (werden häufig auch als Lezithine bezeichnet) und ein Lezithin auch Triglyceride enthalten. Als Öle werden dabei insbesondere im Handel erhältliche Öle eingesetzt, die entschleimt bzw. entlezithiniert sind. Allerdings können auch die nicht behandelten Rohöle je nach Erfordernis zur Anwendung gebracht werden.

Zur Stabilisierung der beanspruchten, hochungesättigten Fettmischung vor autoxidativem Verderb können dem Fachmann bekannte natürliche und synthetische Antioxydantien (wie Ascorbylpalmitat, Tocopherole, etc.) eingesetzt werden. Weiterhin tragen die in der Fettmischung beanspruchten Gehalte an Lezithinen tierischen, pflanzlichen und/oder mikrobiellen Ursprungs zur Oxidationsstabilität derselben bei.

In der nachfolgenden Tabelle 1 sind die Rohstoffe bzw. Fette, Öle und Lezithine aufgeführt, aus denen verschiedene bevorzugte Ausführungsformen der erfindungsgemäßen Fettmischung durch Vermischen hergestellt wurden. In der ebenfalls nachfolgenden Tabelle 2 ist die resultierende Fettsäurezusammensetzung einiger der in der Tabelle 1 aufgeführten Ausführungsbeispiele gezeigt. Der Ausdruck "Blend" ist dabei ein Synonym für den Ausdruck "Mischung".

**Tabelle 1:**

| Zusammensetzung beispielhafter Fettmischungen (Angaben in Gew.-%) | | | | | | |
|---|---|---|---|---|---|---|
| **Rohstoffes** | **Blend A** | **Blend B** | **Blend C** | **Blend D** | **Blend E** | **Blend F** |
| MCT-Fett | 30.0 | 30.0 | 30.0 | - | 30.0 | 30.0 |
| Palm Öl | 26.0 | 16.5 | 20.0 | 26.0 | 26.0 | 26.0 |
| Soyaöl | 16.5 | 11.5 | 8.0 | 16.5 | 17.5 | 13.5 |
| Cocosnuss-Öl | | | | 30.0 | - | |
| Borretschöl | 8.0 | 10.0 | 12.0 | - | - | - |
| Echium-Öl | 11.0 | 13.0 | 18.0 | 19.0 | 19.0 | 19.0 |
| ***Fischöl A*** | - | 16.0 | - | - | - | - |
| ***Fischöl B*** | 6.5 | - | 10.0 | 6.5 | 6.5 | 6.5 |
| Eilipide/Eilezithine | 2.0 | - | 2.0 | 2.0 | 1.0 | 5.0 |
| Fischlezithin | | 3 | | | | |

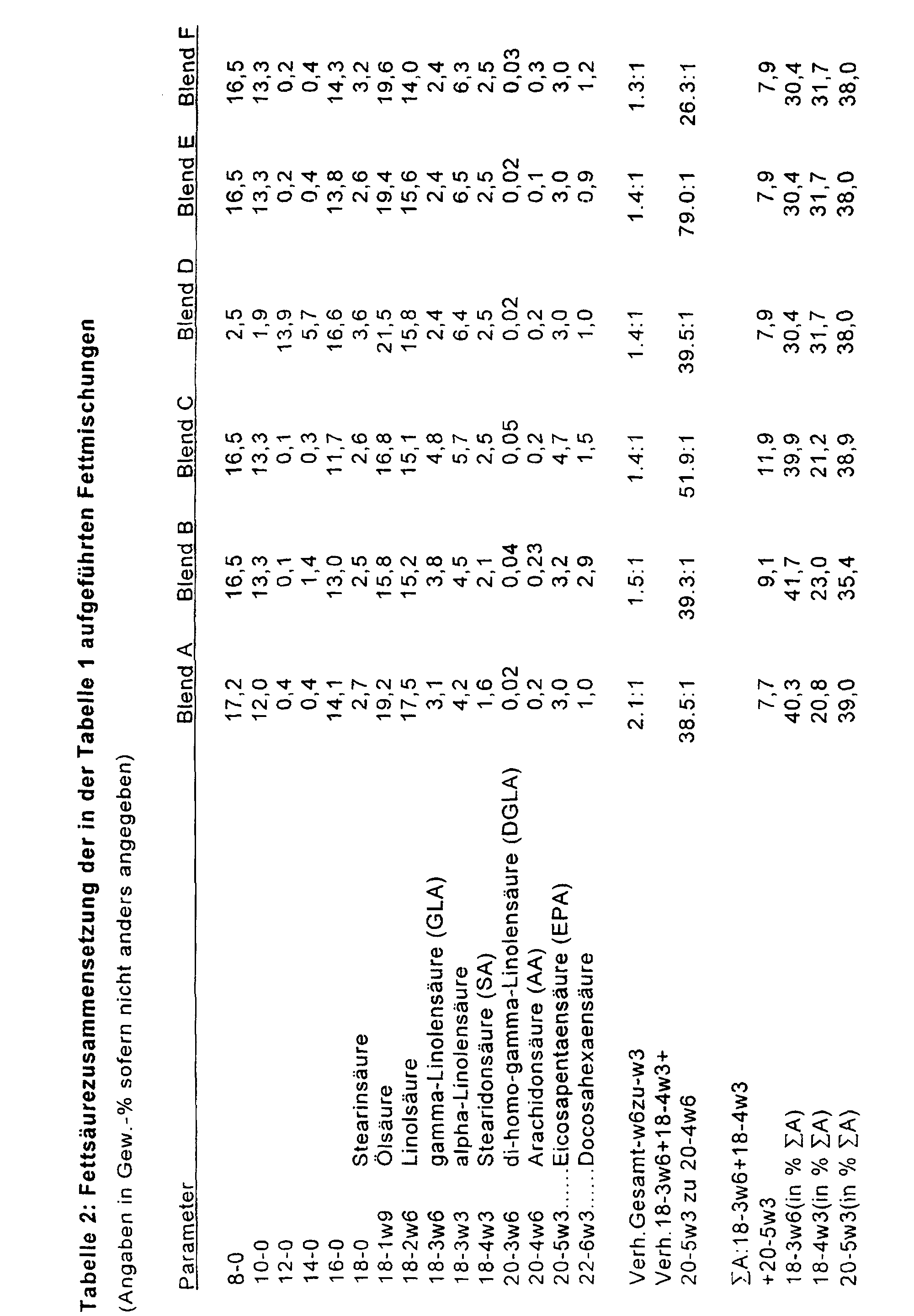

Die erfindungsgemäße Fettmischung kann auch einem diätetischen oder pharmazeutischen Mittel nach dem Stand der Technik einverleibt sein. Dies schließt auch die Verwendung der Fettmischung selbst oder auch von Bestandteilen daraus in mikroverkapselter Form mit ein. Die weiteren Bestandteile dieses Nahrungsmittels bzw. Diätetikums oder Pharmazeutikums können bekannter sowie beliebiger Natur sein und sind den entsprechenden Bedürfnissen angepaßt. Vorzugsweise handelt es sich dabei um eine eine Fettmulsion, eine Fertignahrung, eine Flüssignahrung, eine rekonstituierte Pulvernahrung oder eine zu rekonstituierende Pulvernahrung. Diese Nahrungen dienen insbesondere zur pareneralen, enteralen und/oder oralen Verabreichung. Es kann sich jedoch auch um einen Nahrungsriegel oder um eine streichfähige Paste handeln.

Die erfindungsgemäßen Flüssignahrungen und zu rekonstituierenden Pulvernahrungen dienen insbesondere zur parenteralen, enteralen und/oder oralen Ernährung und weisen vorzugsweise einen Fettgehalt auf, der 10 bis 55 Energie-% beträgt; die Energiedichte beträgt vorzugsweise 0,5 bis 3,0 kcal/ml. Der Fettgehalt macht weiterhin insbesondere bevorzugt 25 bis 40 Energie-% aus, während die Energiedichte insbesondere bevorzugt 1,1 - 1,4 kcal/ml beträgt.

Die erfindungsgemäßen Flüssignahrungen und zu rekonstituirenden Pulvernahrungen sind dadurch gekennzeichnet, daß die Fettsäuren gama-Linolensäure stearidonsäure und Eicosapentasäure zusammen 0,5 bis 30 g/1500 ml (bzw. 1 bis 10 g/1500 ml der flüssigen Nahrung ausmachen.

Die erfindungsgemäßen diätetischen Nahrungsmittel enthalten nicht nur eine Fettmischung bzw. einen Fettblend der hier beschriebenen Art sondern können auch andere Produkte, beispielsweise Eiweiß tierischen und/oder pflanzlichen Ursprungs, z.B. Milch, Molke, Erbsen, Weizen und/oder Soja, in Form von komplexem und/oder hydrolysiertem Eiweiß, mit oder ohne Zusatz von freien Aminosäuren und/oder Dipeptiden sowie Kohlenhydrate (Maltodextrine), Vitamine, Ballaststoffe, Mineralstoffe, Spurenelemente, Cholin, Taurin, Carnitin, Inositol, Nucleotide in unterschiedlichen Mengenanteilen sowie gegebenenfalls Wasser. Diese weiteren Bestandteile können je nach Belieben mit der Fettmischung vermengt werden.

In der nachfolgenden Tabelle 3 sind die Lipid- und Fettsäuregehalte einiger erfindungsgemäßer Fettmischungen aufgeführt, die in Flüssignahrungen inkorporiert sind. In der Tabelle 4 sind die Werte für die Zusammensetzungen von verschiedenen erfindungsgemäßen Flüssignahrungen aufgeführt. In Tabelle 5 sind Beispielrezepturen für erfindungsgemäße Fettemulsionen angegeben.

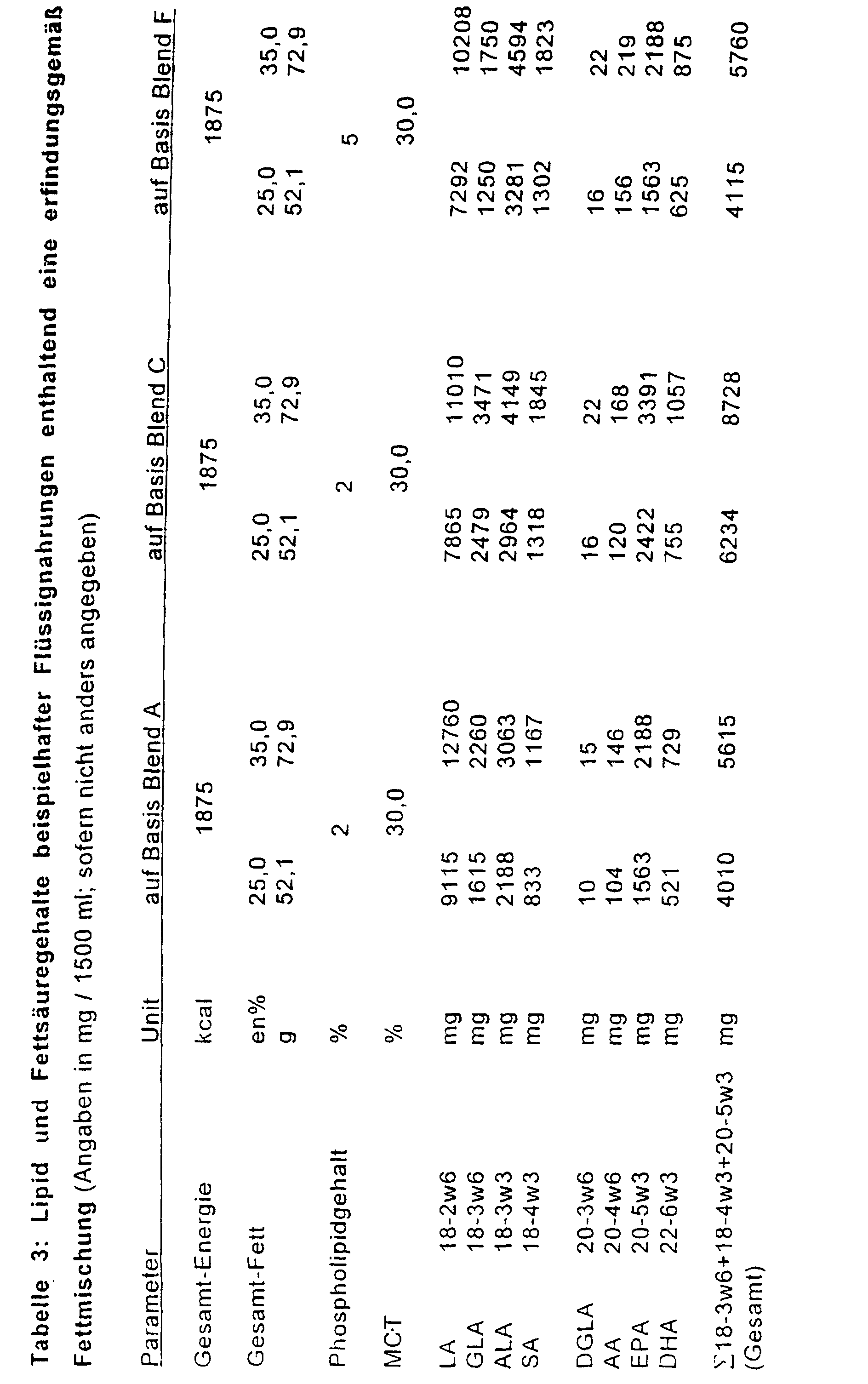

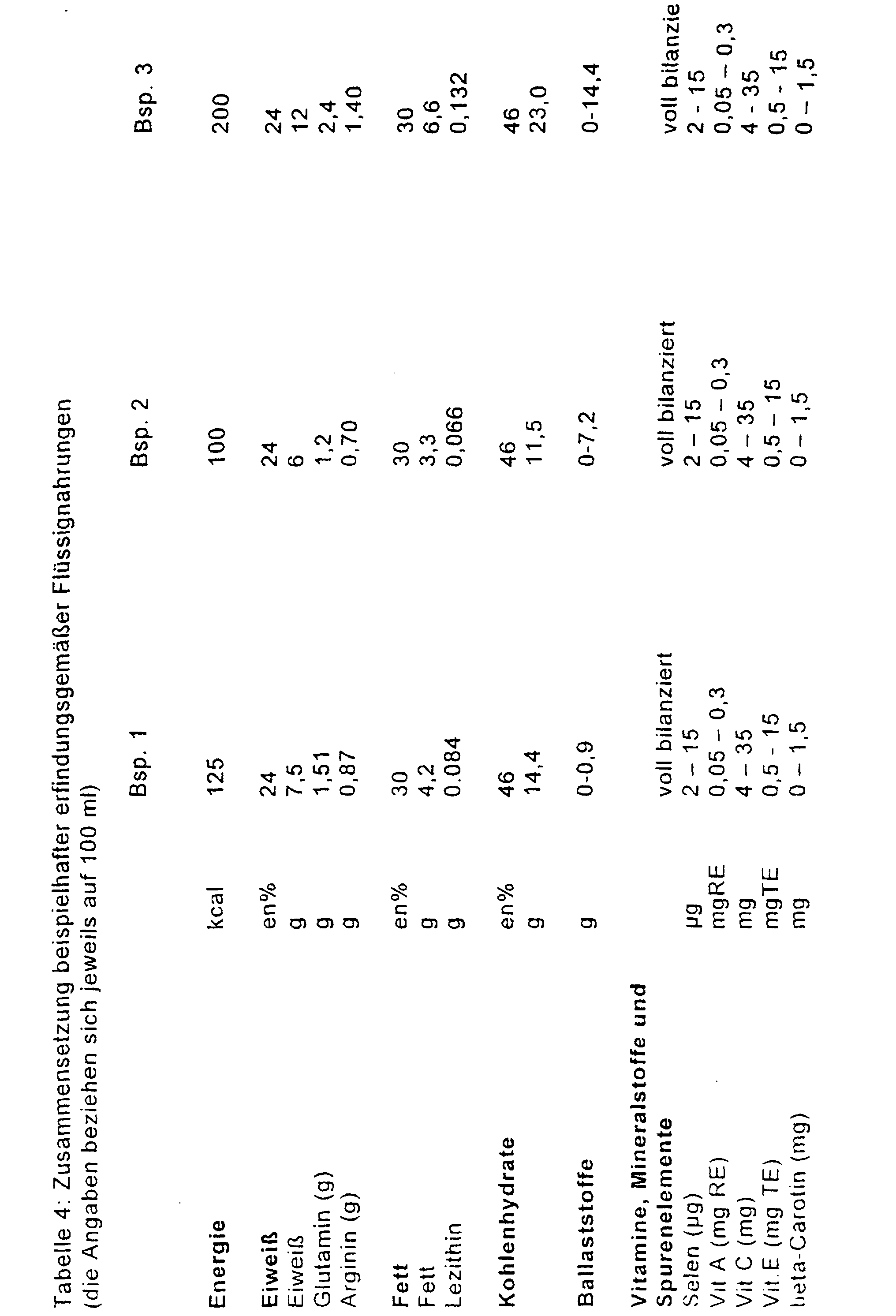

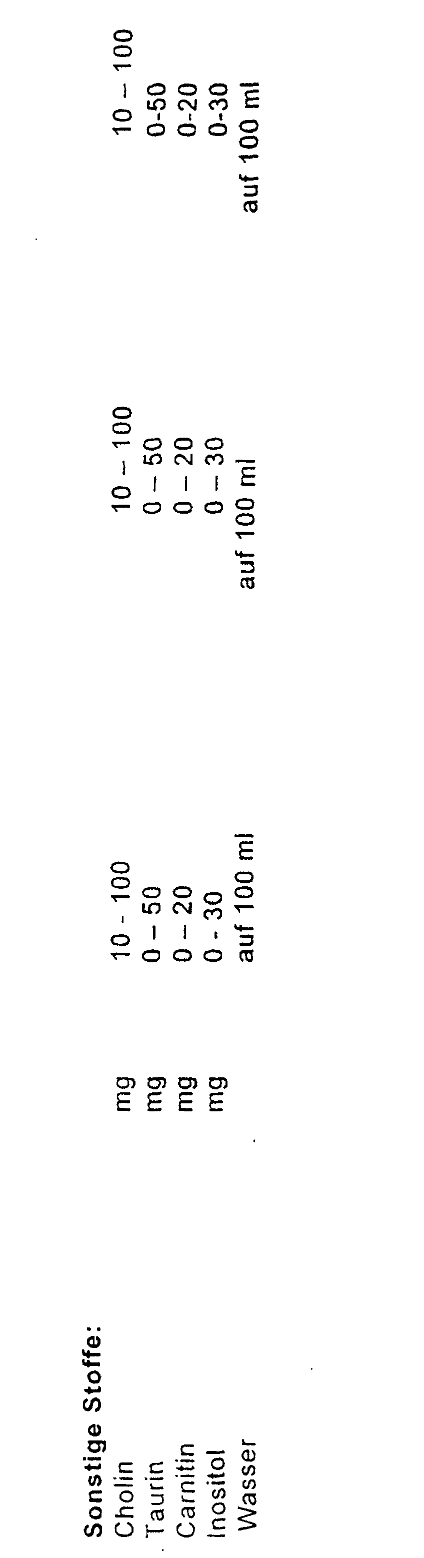

**Tabelle 5:**

| Ausführungsbeispiele der beanspruchten Fettemulsionen (Angaben in g / 100 ml) | | | |
|---|---|---|---|
| Gehalt | Gehalt | Bestandteile | %-Verteilung |
| 3,0 | 6,0 | MCT-Fett | 30% |
| 3,0 | 6,0 | Canola-ÖI | 30 % |
| 1,2 | 2,4 | Fischöl B (45/10) | 12% |
| 1,8 | 3,6 | Borretschöl | 18% |
| 1,0 | 2,0 | Echiumöl | 10% |
| Summe: | Summe: | | Summe: |
| 10 | 20 | Öle und Fette | 100% |
| 1,2 | 1,2 | Eilezithin | |
| 2,25 | 2,25 | Glycerol USP | |
| Bis 100 ml | bis 100 ml | Wasser (zur Injektion) | |

Die erfindungsgemäße Fettmischung und das dieses enthaltende erfindungsgemäße diätetische oder pharmazeutische Mittel kann insbesondere zur Behandlung von Patienten mit den nachgeführten Krankheitszuständen eingesetzt werden:
1. Patienten mit akut- und chronisch inflammatorischen Erkrankungen, mit Autoimmunerkrankungen und mit geschwächter Immunfunktion: z. B. Patienten mit Morbus Crohn, Psoriasis, chronischer Polyarthritis, Rheuma; Patienten mit neurodegenerativne Erkrankungen, Patienten mit pulmonaren Erkrankungen, Patienten in der postoperativen Phase, HIV/AIDS-Patienten, Tumorpatienten, Patienten mit zystischer Fibrose, septeische Patienten, Hochrisikopatienten (infektionsgefährdet, zur Vermeidung/Reduktion von nosokomialen Infektionen), kritisch Kranken (z.B. Polytraume, posttraumatisch, Postagressions-Stoffwechsel, metabolischer Streß), bei Patienten mit generalisiertem Entzündungssyndrom (SIRS: "systemic inflammatory response syndrome"), Multiorganversagen und/oder zur Vorbeugung derselben; bei Koronar-Patienten nach Angioplastie bzw. Bypass-Operation (restenosis, graft occlusion) zur Unterstützung der Immunsuppressionstherapie bei Patienten nach Organtransplantationen und bei Diabetikern.
2. Patienten mit Lipidstoffwechselstörungen:
   z. B. Patienten mit kardiovaskulären Erkrankungen, Hyperlipidämien, Metabolischem Syndrom, u.a.
3. Patienten mit eingeschränkter lipolytischer Kapazität des Gastrointestinaltraktes
   z.B. Patienten mit Morbus Crohn, Colitis ulcerosa, genetische (Cystische Fibrose), Shwachman-Syndrom) entwicklungsbedingter (Neugeborene) oder erworbener exokriner Pankreasinsuffizienz, mit Kurzdarmsyndrom bzw. durch Strahlen oder Zytostatika geschädigtem Gastrointestinaltrakt, nach akuter oder chronisch vollständig parenteraler Ernährung sowie Patienten mit Erkrankungen der Leber und Gallenwege (chronische Hepatitis; Alkohol-Syndrom, Fettleber).

## Patentansprüche

1. Fettmischung, aufgebaut aus den Bestandteilen, die ausgewählt sind aus der Gruppe bestehend aus Ölen, Fetten, Lezithinen, Fettsäuren und deren Salzen und Estern, und enthaltend mehrfach ungesättigte Fettsäuren,
**dadurch gekennzeichnet,**
**daß** die Fettsäuren gamma-Linolensäure, Stearidonsäure und Eicosapentaensäure zusammen 10 bis 500 mg/g Gesamtfettsäuren ausmachen und
die gamma-Linolensäure und die Eicosapentaensäure jeweils 20 bis 50 Gew.-% und die Stearidonsäure 15 bis 50 Gew.-% der Summe dieser drei Fettsäuren ausmachen.

2. Fettmischung nach Anspruch 1,
**dadurch gekennzeichnet,**
**daß** die Fettsäuren gamma-Linolensäure, Stearidonsäure und Eicosapentaensäure zusammen 10 bis 100 mg/g Gesamtfettsäuren ausmachen und
die gamma-Linolensäure und die Eicosapentaensäure jeweils 35 bis 45 Gew.-% und die Stearidonsäure 15 bis 25 Gew.-% der Summe dieser drei Fettsäuren ausmachen.

3. Fettmischung nach Anspruch 2,
**dadurch gekennzeichnet,**
**daß** die gamma-Linolensäure und die Eicosapentaensäure jeweils 40 Gew.-% und die Stearidonsäure 20 Gew.-% der Summe dieser drei Fettsäuren ausmachen.

4. Fettmischung nach einem der vorhergehenden Ansprüche,
**dadurch gekennzeichnet,**
**daß** sie Arachidonsäure enthält und daß der Quotient aus der Summe der gamma-Linolensäure plus Stearidonsäure plus Eicosapentaensäure zur Arachidonsäure mindestens 10:1 beträgt.

5. Fettmischung nach einem der vorhergehenden Ansprüche,
**dadurch gekennzeichnet,**
**daß** der Gehalt an Phospholipiden bis zu 40 Gew.-% der Gesamtlipide (= Summe der Öle, Fette und Lezithine) beträgt.

6. Fettmischung nach Anspruch 5,
**dadurch gekennzeichnet,**
**daß** die Phospholipide 1 bis 10 Gew.-% der Gesamtlipide ausmachen.

7. Fettmischung nach einem der vorhergehenden Ansprüche,
**dadurch gekennzeichnet,**
**daß** die Summe der in der Fettmischung in Form von Phospholipiden vorliegenden Fettsäuren gamma-Linolensäure, Stearidonsäure und Eicosapentaensäure bis zu 120 mg/g Gesamtfettsäuren ausmachen.

8. Fettmischung nach einem der vorhergehenden Ansprüche,
**dadurch gekennzeichnet,**
**daß** die Summe der in der Fettmischung in Form von Phospholipiden vorliegenden Fettsäuren gamma-Linolensäure, Stearidonsäure und Eicosapentaensäure 0,05 - 50 mg/g Gesamtfettsäuren ausmachen.

9. Fettmischung nach einem der vorhergehenden Ansprüche zur parenteralen, enteralen und/oder oralen Verabreichung an Patienten mit chronisch-inflammatorischen Erkrankungen, mit Lipidstoffwechselstörungen, mit geschwächter Immunfunktion und/oder mit eingeschränkter lipolytischer Kapazität des Gastrointestinaltraktes.

10. Diätetisches oder pharmazeutisches Mittel enthaltend eine Fettmischung nach einem der vorherigen Ansprüche 1 bis 8.

11. Mittel nach Anspruch 10,
**dadurch gekennzeichnet,**
**daß** es sich um eine Fettemulsion, eine Fertignahrung, eine Flüssignahrung, eine rekonstituierte oder zu rekonstituierende Pulvernahrung, insbesondere zur parenteralen, enteralen und/oder oralen Verabreichung, um einen Nahrungsriegel oder um eine streichfähige Paste handelt.

12. Mittel nach Anspruch 11 in Form einer Flüssignahrung oder rekonstituierten Pulvernahrung zur parenteralen, enteralen und/oder oralen Ernährung,
**dadurch gekennzeichnet,**
**daß** der Fettgehalt 10 bis 55 Energie-% ausmacht und die Energiedichte 0,5 bis 3,0 kcal/ml beträgt.

13. Mittel nach Anspruch 12,
**dadurch gekennzeichnet,**
**daß** der Fettgehalt 25 bis 40 Energie-% ausmacht und die Energiedichte 1,1 - 1,4 kcal/ml beträgt.

14. Mittel nach einem der Ansprüche 10 bis 13 in Form einer Flüssignahrung oder rekonstituierten Pulvernahrung,
**dadurch gekennzeichnet,**
**daß** die Fettsäuren gamma-Linolensäure, Stearidonsäure und Eicosapentaensäure zusammen 0,5 bis 30 g/1500 ml der flüssigen Nahrung ausmachen.

15. Mittel nach Anspruch 14,
**dadurch gekennzeichnet,**
**daß** die gamma-Linolensäure, Stearidonsäure und Eicosapentaensäure zusammen 1 bis 10 g/1500 ml der flüssigen Nahrung ausmachen.

## Claims

1. Fat blend, built up from the components which are selected from the group consisting of oils, fats, lecithins, fatty acids and salts and esters thereof, and containing polyunsaturated fatty acids,
**characterised in that** the fatty acids gamma-linolenic acid, stearidonic acid and eicosa-pentaenoic acid together comprise 10 to 500 mg/g total fatty acids and
the gamma-linolenic acid and the eicosapentaenoic acid each comprise 20 to 50 wt. % and the stearidonic acid 15 to 50 wt. % of the sum of these three fatty acids.

2. Fat blend according to Claim 1,
**characterised in that** the fatty acids gamma-linolenic acid, stearidonic acid and eicosa-pentaenoic acid together comprise 10 to 100 mg per g total fatty acids and
the gamma-linolenic acid and the eicosapentaenoic acid each comprise 35 to 45 wt. % and the stearidonic acid 15 to 25 wt. % of the sum of these three fatty acids.

3. Fat blend according to Claim 2,
**characterised in that** the gamma-linolenic acid and the eicosapentaenoic acid each comprise 40 wt. % and the stearidonic acid 20 wt. % of the sum of these three fatty acids.

4. Fat blend according to one of the foregoing Claims,
**characterised in that** it contains arachidonic acid and that the quotient of the sum of the gamma-linolenic acid plus stearidonic acid plus eicosapentaenoic acid to the arachidonic acid is at least 10:1.

5. Fat blend according to one of the foregoing Claims,
**characterised in that** the content of phospholipids is up to 40 wt. % of the total lipids
(= sum of the oils, fats and lecithins).

6. Fat blend according to Claim 5,
**characterised in that** the phospholipids comprise 1 to 10 wt. % of the total lipids.

7. Fat blend according to one of the foregoing Claims,
**characterised in that** the sum of the fatty acids gamma-linolenic acid, stearidonic acid and eicosapentaenoic acid present in the fat blend in the form of phospholipids comprises up to 120 mg/g total fatty acids.

8. Fat blend according to one of the foregoing Claims,
**characterised in that** the sum of the fatty acids gamma-linolenic acid, stearidonic acid and eicosapentaenoic acid present in the fat blend in the form of phospholipids comprises 0.05 to 50 mg/g total fatty acids.

9. Fat blend according to one of the foregoing Claims for parenteral, enteral and/or oral administration to patients with chronic inflammatory diseases, with lipid metabolism disorders, with weakened immune function and/or with limited lipolytic capacity of the gastrointestinal tract.

10. Dietetic or pharmaceutical composition containing a fat blend according to one of the foregoing Claims 1 to 8.

11. Composition according to Claim 10,
**characterised in that** it is a fat emulsion, a ready-for-use food, a liquid food, a reconst-ituted or reconstitutable powder food, in particular for parenteral, enteral and/or oral administration, a food strip or a spreadable paste.

12. Composition according to Claim 11 in the form of a liquid food or reconstituted powder food for parenteral, enteral and/or oral feeding,
**characterised in that** the fat content is 10 to 55 energy % and the energy density is 0.5 to 3.0 kcal/ml.

13. Composition according to Claim 12,
**characterised in that** the fat content is 25 to 40 energy % and the energy density is 1.1 to 1.4 kcal/ml.

14. Composition according to one of Claims 10 to 13 in the form of a liquid food or reconstituted powder food,
**characterised in that** the fatty acids gamma-linolenic acid, stearidonic acid and eicosapentaenoic acid together comprise 0.5 to 30 g/1500 ml of the liquid food.

15. Composition according to Claim 14,
**characterised in that** the gamma-linolenic acid, stearidonic acid and eicosapentaenoic acid together comprise 1 to 10 g/1500 ml of the liquid food.

## Revendications

1. Mélange de corps gras formé de composants choisis dans l'ensemble constitué d'huiles, de graisses, de lécithines, d'acides gras et de leurs sels et esters, et contenant des acides gras poly-insaturés, **caractérisé par le fait que** les acides gras gamma-linolénique, stéaridonique et eicosapentaénique constituent ensemble de 10 à 500 mg/g de la totalité des acides gras, et que l'acide gamma-linolénique et l'acide eicosapentaénique constituent chacun de 20 à 50 % en poids et l'acide stéaridonique constitue de 15 à 50 % en poids de la somme de ces trois acides gras.

2. Mélange de corps gras selon la revendication 1, **caractérisé par le fait que** les acides gras gamma-linolénique, stéaridonique et eicosapentaénique constituent ensemble de 10 à 100 mg/g de la totalité des acides gras, et que l'acide gamma-linolénique et l'acide eicosapentaénique constituent chacun de 35 à 45 % en poids et l'acide stéaridonique constitue de 15 à 25 % en poids de la somme de ces trois acides gras.

3. Mélange de corps gras selon la revendication 2, **caractérisé par le fait que** l'acide gamma-linolénique et l'acide eicosapentaénique constituent chacun 40 % en poids et l'acide stéaridonique constitue 20 % en poids de la somme de ces trois acides gras.

4. Mélange de corps gras selon l'une quelconque des revendications précédentes, **caractérisé par le fait qu'**il contient de l'acide arachidonique et que le rapport de la somme des acides gamma-linolénique, stéaridonique et eicosapentaénique à l'acide arachidonique est d'au moins 10 :1 .

5. Mélange de corps gras selon l'une quelconque des revendications précédentes, **caractérisé par le fait que** la teneur en phospholipides constitue jusqu'à 40 % en poids de la teneur totale en lipides (= la somme des huiles, graisses et lécithines).

6. Mélange de corps gras selon la revendication 5, **caractérisé par le fait que** les phospholipides constituent de 1 à 10 % en poids de la teneur totale en lipides.

7. Mélange de corps gras selon l'une quelconque des revendications précédentes, **caractérisé par le fait que** la somme des acides gras présents dans le mélange de corps gras sous la forme de phospholipides, l'acide gamma-linolénique, l'acide stéaridonique et l'acide eicosapentaénique constituent jusqu'à 120 mg/g de la totalité des acides gras.

8. Mélange de corps gras selon l'une quelconque des revendications précédentes, **caractérisé par le fait que** la somme des acides gras présents dans le mélange de corps gras sous la forme de phospholipides, l'acide gamma-linolénique, l'acide stéaridonique et l'acide eicosapentaénique constituent jusqu'à 0,05-50 mg/g de la totalité des acides gras.

9. Mélange de corps gras selon l'une quelconque des revendications précédentes, destiné à une administration par voie parentérale, entérale et/ou orale à des patients atteints de maladies inflammatoires chroniques, de troubles du métabolisme lipidique, de déficience immunitaire et/ou dont le tractus gastro-intestinal présente une capacité lipolytique réduite.

10. Produit diététique ou pharmaceutique contenant un mélange de corps gras selon l'une quelconque des revendications 1 à 8.

11. Produit selon la revendication 10, **caractérisé par le fait qu'**il s'agit d'une émulsion grasse, d'un aliment prêt à la consommation, d'un aliment liquide, d'un aliment en poudre reconstitué ou d'un aliment en poudre à reconstituer, destinés en particulier à une administration par voie parentérale, entérale et/ou orale, d'un aliment en forme de pain ou d'une pâte à tartiner.

12. Produit selon la revendication 11 sous la forme d'un aliment liquide ou d'un aliment en poudre reconstitué destiné à une administration par voie parentérale, entérale et/ou orale, **caractérisé par le fait que** la teneur en matières grasses correspond à 10 - 55 % d'énergie et que la densité d'énergie correspond à 0,5 - 3,0 kcal/ml.

13. Produit selon la revendication 12, **caractérisé par le fait que** la teneur en matières grasses correspond à 25 - 40 % d'énergie et que la densité d'énergie correspond à 1,1-1,4 kcal/ml.

14. Produit selon l'une quelconque des revendications 10 à 13 sous la forme d'un aliment liquide ou d'un aliment en poudre reconstitué, **caractérisé par le fait que** les acides gras gamma-linolénique, stéaridonique et eicosapentaénique constituent ensemble de 0,5 à 30 g/1500 ml de l'aliment liquide.

15. Produit selon la revendication 14, **caractérisé par le fait que** les acides gras gamma-linolénique, stéaridonique et eicosapentaénique constituent ensemble de 1 à 10 g/1500 ml de l'aliment liquide.
